# EUROPÄISCHE PATENTANMELDUNG

(11) **EP 1 479 354 A1**
(43) Veröffentlichungstag der Anmeldung: **24.11.2004**
(21) Anmeldenummer: 03011555.4
(22) Anmeldetag: 21.05.2003
(51) Int. Cl.: A61B 19/00

(54) **Marker zur Positionserfassung mittels Kernspin**

(71) Anmelder: Prohealth AG, 81379 München (DE)
(72) Erfinder: Rinecker, Hans, Dr., 81379 München (DE); Hauffe, Jörg, 81541 München (DE)
(74) Vertreter: Strobel, Wolfgang, Dipl.-Ing.

(57) **Zusammenfassung**

Die Erfindung betrifft die Verwendung eines magnetisch nicht anregbaren Materials zur Herstellung eines diagnostischen, im menschlichen Körper anbringbaren Positionsmarkierungsmittels (2) zur Verwendung bei einer am lebenden Objekt durchgeführten MRT-Abbildung zur relativen Positionserfassung von im menschlichen Körper befindlichen Punkten, wobei dieses Positionsmarkierungsmittel (2) einen Durchmesser von bevorzugt zwischen 0,5 und 4,0 mm aufweist, und wobei das magnetisch nicht anregbare Material hinsichtlich seiner antimagnetischen Eigenschaften so beschaffen ist, daß das Mittel (2) durch magnetisch negative Abbildung als "schwarzes Loch" (4) in der MRT-Abbildung lokalisiert werden kann, ohne erhebliche Artefakte in der Umgebung zu erzeugen, und wobei das Material hinsichtlich seiner Dichteeigenschaften derart beschaffen ist, daß es auch in einer CT- bzw. Röntgenaufnahme deutlich erkennbar ist.

## Beschreibung

Die Erfindung betrifft die Verwendung eines magnetisch nicht anregbaren Materials zur Herstellung eines diagnostischen, im menschlichen Körper anbringbaren Positionsmarkierungsmittels zur Verwendung bei einer MRT (Kernspintomographie) -Abbildung.

Die MRT-Technologie ist ein weitverbreitetes Verfahren zur bildlichen Darstellung von Körperregionen. Es liefert nicht nur Informationen über den Wassergehalt eines speziellen Gewebes, sondern gegebenenfalls auch über die chemische Gewebestruktur, die bei normalem und patologischem Gewebe unterschiedlich ist.

Beim MRT-Verfahren wird ein starkes und homogenes Magnetfeld auf das zu untersuchende Gewebe angewendet. Durch Studium der sogenannten Relaxationszeiten der Protonen der vorhandenen Moleküle, insbesondere der Protonen von Wasser, ist es möglich, über aufwendige und komplexe Computerberechnungen eine bildliche Darstellung der Struktur des betroffenen Gewebes zu erhalten.

MRT-Abbildungen werden auch zur Vorbereitung der Therapieplanung im Bereich moderner Krebsbestrahlungsmedizin angewendet. Hierbei werden Tumore lokalisiert und in ein Referenzsystem eingebettet, das meist aus knöchernen Fixpunkten (Landmarken) des menschlichen Skeletts besteht. Wenn die örtliche Orientierung dieser Fixpunkte zum Tumor ermittelt wurde, kann kurz vor der eigentlichen Bestrahlung eine einfache Überprüfung der Patientenpositionierung erfolgen, indem mittels einer Standardröntgeneinrichtung eine Aufnahme vom Skelett des Patienten im gewünschten Bereich gemacht wird und die erhaltenen Fixpunkte mit den zuvor ermittelten Fixpunkten verglichen werden. Wenn hier eine Übereinstimmung vorliegt, ist klar, daß sich der Tumor an der Stelle befindet, für die die Therapieplanung berechnet wurde, so daß bei der Bestrahlung keine Schädigung des umliegenden Gewebes auftritt.

Schwieriger ist dies bei der gewünschten Bestrahlung von Augen, die aufgrund ihrer Beweglichkeit den Einsatz knöcherner Fixpunkte verhindern. Um auch hier ein sinnvolles Referenzsystem zu erhalten, das mittels Röntenstrahlung detektierbar ist, müssen im Auge, vorzugsweise nahe der Umschlagfalte des Bindehautsackes, an der Sklera Elemente angebracht werden, die sowohl im Kernspinbild als auch im Röntgenbild bzw. CT-Bild sichtbar sind.

Derartige Materialien sind schwer zu finden, da sehr dichte Materialien, die in konventionellen Röntgenbildern gut sichtbar sind, die Kernspinaufnahme stören, indem sie magnetische Eigenschaften aufweisen, die die umliegenden Bereiche des menschlichen Körpers beeinflussen, wodurch Umgebungsartefakte auf der Abbildung entstehen, die sich über einen großen Bereich ausdehnen können.

Es ist daher die Aufgabe der vorliegenden Erfindung, bei der Herstellung eines Positionsmarkierungsmittels zur Verwendung in der Kernspintechnik ein Material zu verwenden, das in allen möglichen Abbildungsarten ohne Einschränkungen verwendet werden kann.

Diese Aufgabe wird durch die Merkmale des Anspruchs 1 gelöst.

Erfindungsgemäß wird ein magnetisch nicht anregbares Material zur Herstellung eines diagnostischen, im menschlichen Körper anbringbaren Positionsmarkierungsmittels zur Verwendung bei einer am lebenden Objekt durchgeführten MRT-Abbildung zur relativen Positionserfassung von im menschlichen Körper befindlichen Punkten verwendet, wobei dieses Positionsmarkierungsmittel einen Durchmesser von bevorzugt zwischen 0,5 und 4,0 mm aufweist, und wobei das magnetisch nicht anregbare Material hinsichtlich seiner antimagnetischen Eigenschaften derart beschaffen ist, daß das Mittel durch magnetisch negative Abbildung als "schwarzes Loch" in der MRT-Abbildung lokalisiert werden kann, ohne erhebliche Artefakte in der Umgebung zu erzeugen, und wobei das Material hinsichtlich seiner Dichteeigenschaften derart beschaffen ist, daß es auch in einer CT- bzw. Röntgenaufnahme deutlich erkennbar ist. Durch diese Universalität eignet sich das Positionsmarkierungsmittel besonders für einen Einsatz bei der Vorbereitung einer Krebsbestrahlungstherapie.

Besonders bevorzugt ist die Verwendung von Titanlegierungen, welche besonders scharfe Kernspinbilder liefern.

Vorzugsweise ist das Positionsmarkierungsmittel als offener Clip ausgebildet, so daß er auch ohne fremde Befestigungsmittel im Körpergewebe befestigbar ist.

Des weiteren ist ein System zur relativen Positionserfasung von im menschlichen Körper befindlichen Punkten sowie ein Verfahren hierzu offenbart.

Weitere Einzelheiten, Merkmale und Vorteile der vorliegenden Erfindung ergeben sich unter Bezugnahme auf die Zeichnungen. Darin zeigt
- Fig. 1: einen Schnitt durch ein Positionsmarkierungsmittel in geöffnetem Zustand, und
- Fig. 2: einen Schnitt durch das Positionsmarkierungsmittel aus Fig. 1 in geschlossenem Zustand.

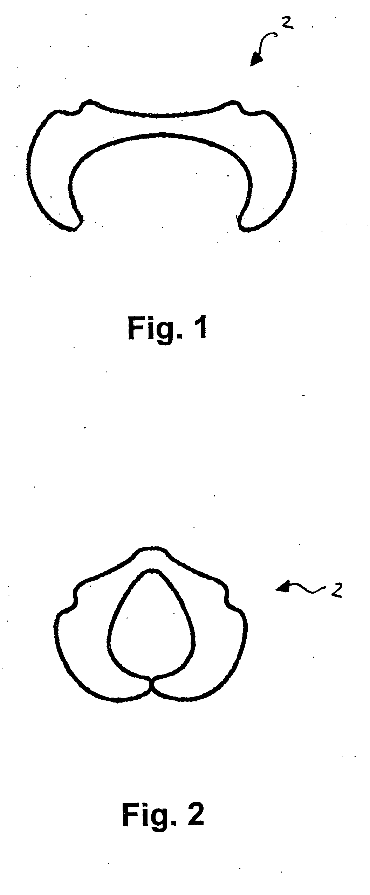

In Fig. 1 ist ein erfindungsgemäßes Positionsmarkierungsmittel 2 dargestellt. Zu seiner Herstellung wird ein antimagnetisches Material verwendet, das gleichzeitig eine derartige Dichteeigenschaft aufweist, daß Röntgenstrahlung nicht hindurchtreten kann. Außerdem sollte das Material derart neutral sein, daß es im menschlichen Körper implantiert werden kann. Hierfür eignet sich in besonderem Maße das leichte Edelmetall Titan und Legierungen hieraus, die beispielsweise bereits in der Gefäßchirurgie verwendet werden. Das Positionsmarkierungsmittel 2 kann jede beliebige Form aufweisen, sollte jedoch nicht zu groß sein, um noch eine genaue Lokalisierung zu ermöglichen. Vorzugsweise ist es als offener Clip mit einer Ausdehnung von zwischen 0,5 mm und 4,0 mm ausgebildet. Der Clip ist durch geeignete Vorrichtungen zu einem geschlossenen ringförmigen Körper zusammenfügbar. Das Positionsmarkierungsmittel 2 zeichnet sich dadurch aus, daß es in Kernspinaufnahmen kleine signalnegative Auslöschhöfe ("schwarze Löcher 4") sowie in CToder Röntgenaufnahmen positive Signale ohne Umgebungsartefakte zeigt. Dadurch eignet es sich hervorragend zu einer Verwendung in einem Verfahren zur relativen Positionserfassung eines Tumors im Augenbereich, der aufgrund der Beweglichkeit des Auges nicht in einem üblichen Bezugssystem anhand knöcherner Landmarken lokalisiert werden kann. Hierzu werden üblicherweise eine Mehrzahl dieser Mittel verwendet, vorzugsweise drei.

Das Verfahren zur relativen Positionserfassung und -verifizierung läuft folgendermaßen ab:

Zunächst wird mittels eines üblichen Kernspintomographen eine Kernspinaufnahme von der betroffenen Körperregion gemacht, wobei die Positionsmarkierungsmittel 2 als "schwarze Löcher" abgebildet werden, die durch ihre antimagnetischen Eigenschaften erklärbar sind. Dabei werden die Zellen in der näheren Umgebung der Positionsmarkierungsmittel nur unwesentlich beeinträchtigt, so daß ein scharfes Bild des gesamten Zielbereichs erzeugt werden kann. Hierbei ist eine Immobilisierung des Patienten sowie die Fixierung eines Auges auf ein Fixationslicht vonnöten. Im Anschluß daran wird kurz vor der eigentlichen Bestrahlung des Patienten eine Röntgenaufnahme des selben Teils des menschlichen Körpers gemacht, in der die Positionsmarkierungsmittel positive Signale geben.

Die Lage der markierten Punkte kann anschließend von einem Arzt mit der ursprünglichen Aufnahme verglichen werden, um festzustellen, ob der zu behandelnde, bewegliche Bereich sich in der richtigen Position und Ausrichtung befindet. Erst dann wird mit der Bestrahlung begonnen.

## Patentansprüche

1. Verwendung eines magnetisch nicht anregbaren Materials zur Herstellung eines diagnostischen, im menschlichen Körper anbringbaren Positionsmarkierungsmittels (2) zur Verwendung bei einer am lebenden Objekt durchgeführten MRT-Abbildung zur relativen Positionserfassung von im menschlichen Körper befindlichen Punkten, wobei dieses Positionsmarkierungsmittel (2) einen Durchmesser von bevorzugt zwischen 0,5 und 4,0 mm aufweist, und wobei das magnetisch nicht anregbare Material hinsichtlich seiner antimagnetischen Eigenschaften derart beschaffen ist, daß das Mittel (2) durch magnetisch negative Abbildung als "schwarzes Loch" (4) in der MRT-Abbildung lokalisiert werden kann, ohne erhebliche Artefakte in der Umgebung zu erzeugen, und wobei das Material hinsichtlich seiner Dichteeigenschaften derart beschaffen ist, daß es auch in einer CT- bzw. Röntgenaufnahme deutlich erkennbar ist.

2. Verwendung eines Materials nach Anspruch 1, **dadurch gekennzeichnet, daß** das Material eine Titanlegierung ist.

3. Verwendung eines Materials nach Anspruch 1 oder 2, **dadurch gekennzeichnet, daß** das Positionsmarkierungsmittel (2) als offener Clip ausgebildet ist, der im Körpergewebe befestigbar ist.

4. System zur relativen Positionserfassung von im menschlichen Körper befindlichen Punkten, mit einem MRT-Gerät und mindestens einem Positionsmarkierungsmittel (2) aus magnetisch nicht anregbarem Material, das einen Durchmesser von bevorzugt zwischen 0,5 und 4,0 mm aufweist, und wobei das nicht anregbare Material hinsichtlich seiner antimagnetischen Eigenschaften so beschaffen ist, daß das Mittel (2) durch magnetisch negative Abbildung als "schwarzes Loch" (4) in der MRT-Abbildung lokalisiert werden kann, ohne erhebliche Artefakte in der Umgebung zu erzeugen, wobei das Material des Positionsmarkierungsmittels (2) hinsichtlich seiner Dichteeigenschaften derart beschaffen ist, daß es auch in einer CT- bzw. Röntgenaufnahme deutlich erkennbar ist.

5. System nach Anspruch 4, **dadurch gekennzeichnet, daß** das Material des Positionsmarkierungsmittels (2) eine Titanlegierung ist.

6. System nach Anspruch 4 oder 5, **dadurch gekennzeichnet, daß** das Positionsmarkierungsmittel (2) als offener Clip ausgebildet ist, der im Körpergewebe befestigbar ist.

7. Verfahren zur relativen Positionserfassung von im menschlichen Körper befindlichen Punkten, bei dem eine MRT-Aufnahme von zumindest einem Teil eines menschlichen Körpers gemacht wird, wobei eine Mehrzahl von vor Beginn des Verfahrens im menschlichen Körper angebrachten Positionsmarkierungsmitteln (2) aus einem magnetisch nicht anregbaren Material, die einen Durchmesser von bevorzugt zwischen 0,5 und 4,0 mm aufweisen, aufgrund ihrer antimagnetischen Eigenschaften in der MRT-Abbildung eine magnetisch negative Abbildung als "schwarzes Loch" (4) liefern, ohne erhebliche Artefakte in der Umgebung zu erzeugen, und bei dem eine Röntgenaufnahme von zumindest diesem Teil des menschlichen Körpers gemacht wird, wobei das Material des Positionsmarkierungsmittels (2) hinsichtlich seiner Dichteeigenschaften derart beschaffen ist, daß es sich auch in der Röntgenaufnahme deutlich abhebt.

8. Verfahren nach Anspruch 7, **dadurch gekennzeichnet, daß** das Material des Positionsmarkierungsmittels (2) eine Titanlegierung ist.

9. Verfahren nach Anspruch 7 oder 8, **dadurch gekennzeichnet, daß** das Positionsmarkierungsmittel (2) als offener Clip ausgebildet ist, der im Körpergewebe befestigbar ist.
